# EUROPÄISCHE PATENTSCHRIFT

(11) **EP 0 984 767 B1**
(45) Veröffentlichungstag und Bekanntmachung des Hinweises auf die Patenterteilung: **23.04.2003**
(21) Anmeldenummer: 98952726.2
(22) Anmeldetag: 21.10.1998
(51) Int. Cl.: A61K 7/155

(54) **WARMWACHS ZUR HAARENTFERNUNG**
HOT WAX FOR HAIR REMOVAL
CIRE CHAUDE POUR L'EPILATION

(30) Priorität: 21.02.1998 DE 19807380
(43) Veröffentlichungstag der Anmeldung: 15.03.2000
(73) Patentinhaber: BRAUN GmbH, 61466 Kronberg (DE)
(72) Erfinder: FINGER, Gerhard, D-61389 Schmitten (DE); SCHMIDT, Susan, D-06803 Greppin (DE)
(86) Internationale Anmeldenummer: EP9806668
(87) Internationale Veröffentlichungsnummer: WO99042081

(56) Entgegenhaltungen:
- CA-A- 895 839
- FR-A- 2 656 524
- GB-A- 2 157 951

## Beschreibung

Die Erfindung betrifft ein Zucker enthaltendes Warmwachs zur Haarentfernung.

Zur Haarentfernung, der sogenannten Epilation, werden zwei unterschiedliche Wachsarten verwendet. Hierbei kommen zum einen öllösliche Wachse und zum anderen wasserlösliche Wachse zum Einsatz. So wird in der GB 2 231 494 eine wasserlösliche Zusammensetzung zum Enthaaren beschrieben, die eine Mischung eines Mono- oder Disaccharidzuckers, wie Sucrose, mit einer verdünnten natürlichen Säure, wie Zitronensäure, umfaßt.

In den bekannten Wachsen kommen unter anderem Fructose, Glucose, Saccharose, Wasser, Zitronensäure und Honig zum Einsatz. In der GB 2157951 zum Beispiel wird eine wasserlösliche Zusammensetzung zum Enthaaren beschrieben, die eine Mischung aus Glucose und Invertzucker, Zitronensäure und Honig umfasst.

Bei den bekannten wasserlöslichen Warmwachsen zeigt sich im Vergleich zu den ölföslichen Warmwachsen des Standes der Technik eine geringere Wirksamkeit hinsichtlich der Haarentfernung. Andererseits erfordern die lipophilen Wachse einen zusätzlichen Reinigungsschritt auf der Haut, weil dort Wachsreste zurückbleiben, die mit Öl entfernt werden müssen.

Die Aufgabe der Erfindung bestand daher darin, ein Warmwachs bereitzustellen, das die geschilderten Nachteile der bekannten Warmwachsarten überwindet.

Die Aufgabe wird durch ein Zucker enthaltendes Warmwachs zur Haarentfernung gelöst, das dadurch gekennzeichnet ist, daß es 0-60 Gew.-% Fructose, 1-50 Gew.-% Polyethylenglykol mit einer Molmasse von 10.000-30.000 g/mol und 20-65 Gew.-% einer Mischung aus Glucose- und Invertzuckersirup umfaßt.

Hierin wirkt die Mischung aus Glucose- und Invertzuckersirup als Klebemittel. Die Fructose fungiert als Geliermittel und dient als solches zur Viskositätserhöhung des Wachses. In gleicher Weise wirkt das Polyethylenglykol, das zusätzlich die Wachsformulierung zum schnelleren Aushärten bringt.

In einer bevorzugten Ausführungsform der Erfindung umfaßt die Mischung aus Glucose- und Invertzuckersirup zusätzlich Honig.

In einer weiteren bevorzugten Ausführungsform der Erfindung umfaßt das Warmwachs 30-60 Gew.-% Fructose, 1-25Gew.-% Polyethylenglykol der genannten Molmasse und 40-60 Gew.-% der Mischung aus Glucose- und Invertzuckersirup.

Bevorzugt ist ein Warmwachs, das im Temperaturbereich zwischen 40 C und 80 C eine Viskosität von 10.000-200.000 mPas, vorzugsweise von 22.000-24.000 mPas, aufweist. Die Viskosität wird bei Testmessungen mit dem Rotationsviskosimeter Viscotester VT 02 bestimmt.

Das erfindungsgemäße, wasserlösliche Wachs kombiniert die gute Epilationsleistung eines öllöslichen Wachses mit der sehr guten Reinigungsmöglichkeit eines wasserlöslichen Wachses.

Ein weiterer Gegenstand der Erfindung besteht in einem Verfahren zur Haarentfernung mittels eines Warmwachses, das dadurch gekennzeichnet ist, daß das erfindungsgemäße Warmwachs erwärmt auf die Haut aufgebracht und mit einem Abzugsstreifen wieder von dieser entfernt wird.

Das Aufbringen des Wachses auf die Haut kann durch ein Heizgerät für Warmwachse erfolgen, z.B. mit einem Rollapplikator mit Temperaturkontrolle.

Der Streifen zum Abziehen des Wachses kann aus einem Material aus Natur- oder Kunstfaser bestehen. Beispiele für Naturfasern sind Baumwolle und Zellstoff, ein Beispiel für Kunstfaser ist Polyester. Das bevorzugte Material für den Abzugsstreifen ist Baumwolle.

Die Einsatztemperatur für das beschriebene Verfahren beträgt 50-90 C, wobei eine Temperatur von 70 C bevorzugt ist.

Das erfindungsgemäße Wachs wird bei der Epilation nahezu vollständig von der Haut entfernt. Ein leichter Klebefilm, der zurückbleiben kann, wird nach der Haarentfernung vorzugsweise mit warmem Wasser problemlos von der Haut abgewaschen. Auch eventuell verbleibende Wachsreste, die von dem Baumwoll-Abzugsstreifen nicht erfaßt wurden, können so entfernt werden. Die Haarentfernung kann folglich auch bei der täglichen Körperreinigung in besonders vorteilhafter Weise ohne zusätzliche Hilfsmittel durchgeführt werden.

Ein weiterer Vorteil besteht darin, daß ein ungleichmäßiges Aufbringen der Wachsschicht die Epilation nicht negativ beeinflußt, da die Formulierung des Wachses nach dem Auftragen sehr zäh bis fest wird. Die Aushärtzeit kann ferner ohne nachteilige Beeinflussung auf das Epilier-Ergebnis variiert werden.

Die Erfindung soll anhand des folgenden Beispiels veranschaulicht werden.

Das Warmwachs umfaßt 51 Gew.-% Fructose, 8 Gew.-% Polyethylenglykol mit einer Molmasse von 20.000 g/mol und 41 Gew-% eines Gemisches aus Glucose- und Invertzuckersirup. Diese Formulierung wurde in einem internen Probanden-Gebrauchstest getestet.

Die zu epilierenden Stellen waren hierbei idealerweise trocken. Wo dies nicht gegeben war, wurde die Haut mit einem Feuchtigkeitstuch, das wenig Alkohol enthielt, vorbehandelt. Anschließend wurde das Wachs bei einer Temperatur von 70 C in Haarwuchsrichtung dünn und einmalig aufgetragen. Die Abzugsstreifen aus Baumwolle wurden anschließend auf das Wachs gedrückt. Dann wurde mit der Hand über die aufgelegten Abzugsstreifen in Haarwuchsrichtung gestrichen, bis das Wachs in das Gewebe eingedrungen war. Dabei wurde das Wachs im Gewebe sichtbar, drang jedoch nicht nach außen.

Anschließend wurden die Baumwollstreifen, die mit Wachs vollgesogen waren, entgegen der Haarwuchsrichtung abgerissen. Die Epilation wurde hierbei als schwacher Schmerz empfunden.

Die epilierten Stellen wurden gereinigt und teilweise mit einer Feuchtigkeits-Lotion eingecremt.

Die Epilationsleistung war bei allen Probanden sehr gut. Die Haare wurden stets gründlich entfernt, so daß das erfindungsgemäße Wachs eine hohe Effektivität aufwies ohne Reinigungsprobleme zu verursachen.

## Patentansprüche

1. Zucker enthaltendes Warmwachs zur Haarentfemung, **dadurch gekennzeichnet, daß** es 0-60 Gew.-% Fructose, 1-50 Gew.-% Polyethylenglykol mit einer Molmasse von 10.000-30.000 g/mol und 20-65 Gew.-% einer Mischung aus Glucose- und Invertzuckersirup umfaßt.

2. Warmwachs nach Anspruch 1, **dadurch gekennzeichnet, daß** die Mischung aus Glucose- und Invertzuckersirup zusätzlich Honig umfaßt.

3. Warmwachs nach Anspruch 1 oder 2, **dadurch gekennzeichnet, daß** es
30-60 Gew.-% Fructose, 1-25 Gew.-% des Polyethylenglykols und
40-60 Gew.-% einer Mischung aus Glucose- und Invertzuckersirup umfaßt.

4. Warmwachs nach einem der Ansprüche 1 bis 3, **dadurch gekennzeichnet, daß** es im Temperaturbereich zwischen 40 C und 80 C eine Viskosität von 10.000-200.000 mPas aufweist.

5. Warmwachs nach Anspruch 4, **dadurch gekennzeichnet, daß** es im Temperaturbereich zwischen 40 C und 80 C eine Viskosität von 22.000-24.000 mPas aufweist.

6. Verfahren zur Haarentfernung mittels eines Warmwachses, **dadurch gekennzeichnet, daß** das Warmwachs nach einem der Ansprüche 1 bis 5 erwärmt auf die Haut aufgebracht und mit einem Abzugsstreifen wieder von dieser entfernt wird.

7. Verfahren nach Anspruch 6, **dadurch gekennzeichnet, daß** das Aufbringen auf die Haut durch ein Heizgerät für Warmwachse erfolgt.

8. Verfahren nach Anspruch 7, **dadurch gekennzeichnet, daß** als Heizgerät ein Rollapplikator mit Temperaturkontrolle eingesetzt wird.

9. Verfahren nach einem der Ansprüche 6 bis 8, **dadurch gekennzeichnet, daß** als Abzugsstreifen ein solcher aus Baumwolle und/oder Zellstoff und/oder Polyester eingesetzt wird.

10. Verfahren nach nach einem der Ansprüche 6 bis 9, **dadurch gekennzeichnet, daß** eine Einsatztemperatur von 50-90 C verwendet wird.

## Claims

1. Warm wax containing sugar for hair removal, **characterized in that** it comprises 0-60% by weight of fructose, 1-50% by weight of polyethylene glycol with a molar mass of 10,000-30,000 g/mol and 20-65% by weight of a mixture of glucose and invert sugar syrup.

2. Warm wax according to Claim 1, **characterized in that** the mixture of glucose and invert sugar syrup additionally comprises honey.

3. Warm wax according to Claim 1 or 2, **characterized in that** it comprises
30-60% by weight of fructose, 1-25% by weight of polyethylene glycol and
40-60% by weight of a mixture of glucose and invert sugar syrup.

4. Warm wax according to one of Claims 1 to 3, **characterized in that** it has a viscosity of 10,000-200,000 mPas in the temperature range between 40 C and 80 C.

5. Warm wax according to Claim 4, **characterized in that** it has a viscosity of 22,000-24,000 mPas in the temperature range between 40 C and 80 C.

6. Method of hair removal using a warm wax, **characterized in that** the warm wax according to one of Claims 1 to 5 is heated, applied to the skin and removed again therefrom using a remover strip.

7. Method according to Claim 6, **characterized in that** application onto the skin takes place by means of a heating device for warm waxes.

8. Method according to Claim 7, **characterized in that** the heating device used is a roll-on applicator with temperature control.

9. Method according to one of Claims 6 to 8, **characterized in that** the remover strip used is one made of cotton and/or cellulose and/or polyester.

10. Method according to according to [sic] one of Claims 6 to 9, **characterized in that** a temperature of 50-90 C is used.

## Revendications

1. Cire chaude d'épilation contenant du sucre, **caractérisée en ce qu'**elle contient de 0 à 60% en poids de fructose, de 1 à 50% en poids de polyéthylène glycol d'une masse molaire de 10.000 à 30.000 g/mole et de 20 à 65% en poids d'un mélange de sirops de glucose et de sucre inverti.

2. Cire chaude selon la revendication 1, **caractérisée en ce que** le mélange de sirops de glucose et de sucre inverti contient en outre du miel.

3. Cire chaude selon la revendication 1 ou 2, **caractérisée en ce qu'**elle contient de 30 à 60% en poids de fructose, de 1 à 25% en poids du polyéthylène glycol et de 40 à 60% en poids d'un mélange de sirops de glucose et de sucre inverti.

4. Cire chaude selon l"une des revendications 1 à 3, **caractérisée en ce qu'**elle présente, dans la plage de température entre 40°C et 80°C, une viscosité de 10.000 à 200.000 mPas.

5. Cire chaude selon la revendication 4, **caractérisée en ce qu'**elle présente, dans la plage de température entre 40°C et 80°C, une viscosité de 22.000 à 24.000 Mpas.

6. Procédé d'épilation au moyen d'une cire chaude, **caractérisé en ce que** la cire chaude selon l'une des revendications 1 à 5 est appliquée à chaud sur la peau et en est retirée au moyen d'une bande dépilatoire.

7. Procédé selon la revendication 6, **caractérisé en ce que** l'application sur la peau est réalisée au moyen d'un appareil chauffant pour cire chaude.

8. Procédé selon la revendication 7, **caractérisé en ce que** l'on utilise comme appareil chauffant un applicateur à roulette avec thermostat.

9. Procédé selon l'une des revendications 6 à 8, **caractérisé en ce que** l'on utilise comme bande dépilatoire une bande de coton et / ou de cellulose et / ou de polyester.

10. Procédé selon l'une des revendications 6 à 9, **caractérisé en ce que** l'on utilise une température de mise en oeuvre de 50 à 90°C.
